# EUROPÄISCHE PATENTSCHRIFT

(11) **EP 2 218 399 B1**
(45) Veröffentlichungstag und Bekanntmachung des Hinweises auf die Patenterteilung: **25.09.2013**
(21) Anmeldenummer: 09002176.7
(22) Anmeldetag: 17.02.2009
(51) Int. Cl.: A61B 5/15

(54) **Wiederverwendungsschutz für Lanzettensystem**
Reuse protection for lancet system
Protection contre la réutilisation pour système de lancettes

(43) Veröffentlichungstag der Anmeldung: 18.08.2010
(60) Teilanmeldung: 13003341.8
(73) Patentinhaber: Roche Diagnostics GmbH, 68298 Mannheim (DE); F.Hoffmann-La Roche AG, 4070 Basel (CH)
(72) Erfinder: Weiss, Thomas, 68307 Mannheim (DE); Keil, Michael, 67071 Ludwigshafen (DE); Gentsch, Susanne, 68519 Viernheim (DE); Forster, Richard, 92269 Fensterbach (DE); Gorshöfer, Andreas, 93149 Nittenau (DE); Wessel, Robert, 29690 Schwarmstedt (DE)
(74) Vertreter: Silber, Anton

(56) Entgegenhaltungen:
- EP-A- 1 997 435
- WO-A-2005/018425
- WO-A-2005/018711
- JP-A- 2005 253 781
- JP-A- 2007 185 499
- US-A- 5 324 303
- US-A1- 2002 120 216
- US-A1- 2003 153 939
- US-A1- 2004 260 325
- US-A1- 2005 038 465
- US-A1- 2006 116 607
- US-A1- 2006 178 600
- US-A1- 2007 009 381
- US-A1- 2008 082 023
- US-A1- 2008 267 822
- US-B1- 6 852 119

## Beschreibung

### Gebiet der Erfindung

Die Erfindung betrifft ein Lanzettensystem in Form eines Lanzettenmagazins, das in einer Stechhilfe verwendet werden kann zur Entnahme von Blut für Diagnosezwecke.

Bei verschiedenen Krankheiten ist es notwendig, das menschliche Blut hinsichtlich eines darin enthaltenen Analyten zu untersuchen. In vielen Fällen reicht es dabei aus, durch Erzeugung einer kleinen Stichwunde dem Körper eine geringe Menge Blut in Form eines Blutstropfens zu entnehmen. Ein besonders wichtiger derartiger Fall ist der Diabetes, bei dem das Blut in regelmäßigen Abständen auf den Glukosegehalt untersucht werden muss. Weitere Blutuntersuchungen können zum Beispiel bezüglich der Gerinnungsparameter, Triglyzeride, HbA1c oder Laktat vorgenommen werden. Zur Erzeugung der erforderlichen Stichwunden werden üblicherweise Blutlanzettenvorrichtungen verwendet, die aus einem Stechgerät und hierzu angepassten auswechselbaren Lanzetten bestehen. In dem Gehäuse des Stechgerätes befindet sich ein Lanzettenhalter, in dem jeweils eine Lanzette auswechselbar eingesetzt werden kann. Beim Einstichvorgang wird der Lanzettenhalter von einem ebenfalls in dem Stechgerät integrierten Lanzettenantrieb schnell in eine Einstichrichtung bewegt, bis die Lanzettespitze aus einer vom vorderen Ende des Stechgerätes vorgesehen Austrittsöffnung austritt und eine kleine Stichwunde in den Körperteil, gegen das das vordere Ende gedrückt wird, erzeugt. Danach wird der Lanzettenhalter mit der Lanzette entgegen der Einstichrichtung zurückbewegt.

Im Laufe der Zeit haben sich kleine handliche Blutentnahmegeräte, sogenannte Stechhilfen, etabliert, die vom Benutzer einfach und zuverlässig bedient werden können und einen möglichst schmerzarmen Einstich in ein Körperteil zu ermöglichen. Zur Vermeidung von Infektionen, insbesondere im Krankenhaus, sind die Lanzetten zur einmaligen Verwendung vorgesehene disposible Elemente. Nach der einmaligen Verwendung einer Lanzette wird die Lanzette nach dem Stechvorgang entnommen oder vom Gerät ausgeworfen und in den Müll verworfen. Durch die in einem solchen Fall in einem Müllcontainer freiliegende Lanzette kann es bei der Beseitigung des Abfalls zu Verletzungen kommen, die zur Kontamination Dritter durch die benutzte Lanzette führen können. Eine derartige Kontamination kann unter Umständen Infektionen nach sich ziehen, so dass es in einigen Ländern Bestrebungen gibt, Blutentnahmesysteme, bei denen die Lanzettespitze nach der Benutzung frei zugänglich ist, zu verbieten. Zusätzlich zu einer Verletzungsgefahr bei der Müllbeseitigung besteht darüber hinaus das Risiko einer versehentlichen Wiederverwendung einer bereits gebrauchten Lanzette. Dies ist insbesondere im Krankenhausbereich, bei dem eine Stechhilfe für mehrere Patienten verwendet wird, zu bedenken, da so durch eine Unachtsamkeit des Pflegepersonals ein Patient mit dem Blut eines vorhergehenden Patienten kontaminiert werden kann.

Neben der Verwendung der Blutlanzettenvorrichtung durch medizinisches Personal werden darüber hinaus Stechhilfen auch von Laien im sogenannten Home-Monitoring-Bereich eingesetzt. Dies gilt insbesondere für die Therapiekontrolle von Diabetikern. So wurde bei der Behandlung von Diabetikern festgestellt, dass schwerwiegende, mit dem Diabetes verbundene Schäden, wie zum Beispiel Erblinden, entscheidend vermindert werden können, wenn die Glukosekonzentration im Blut des Diabetikers häufig, bis zu fünf mal täglich, bestimmt wird und aufgrund dieser Messungen die Insulininjektion exakt eingestellt werden kann. Um derart häufige Messungen zu realisieren, werden Stechhilfen im Rahmen des Home-Monitoring-Bereiches eingesetzt, sodass der Diabetiker selbst eine Blutuntersuchung vornehmen kann. Die Anforderungen, die sich hierdurch an eine Blutlanzettenvorrichtung ergeben, sind neben einer einfachen Handhabung beim Auslösen des Stechvorgangs sowie eines schmerzarmen Einstiches auch ein einfaches Handling beim Einsetzen neuer Lanzetten sowie ein sicheres Verwerfen von bereits gebrauchten Lanzetten. Ein Wechsel der Lanzetten sollte einerseits möglichst einfach sein, andererseits aber auch ein Maximum an Sicherheit gegen ungewollte Verletzungen des Anwenders sowie Dritter gewährleisten. Hierbei ist es im Home-Monitoring-Bereich denkbar, dass eine einmal eingelegte Lanzette durch den selben Benutzer zwar mehrmals zum Stechen verwendet wird; jedoch sollte auch hier, nachdem der Benutzer sich entschieden hat, die Lanzette zu verwerfen, eine versehentliche Wiederverwendung einer bereits ausgeworfenen Lanzette verhindert werden. Darüber hinaus sollte ein Schutz vor ausgeworfenen Lanzetten insbesondere für Dritte, beispielsweise bei der Müllbeseitigung, gewährleistet sein.

Im Stand der Technik wird beim Einsetzen der Lanzette die Spitze der Lanzette üblicherweise von einer Spitzenabdeckung aus Kunststoff umgeben, die eine gefahrlose Handhabung beim Einführen der Lanzette ermöglicht. Wenn die Lanzette eingesteckt ist, wird die Spitzenabdeckung entfernt, damit die scharfe Spitze der Lanzette für den Stichvorgang frei liegt (US 5,628,765). Durch die freiliegende Lanzettespitze besteht jedoch sowohl das Risiko einer unbeabsichtigten Verletzung als auch die Möglichkeit einer Spitzenbeschädigung. Nach einem oder mehrmaligem Stechvorgang wird die Lanzette aus der Stechhilfe entfernt. Dies kann entweder manuell geschehen, wobei jedoch das Risiko einer Verletzung an der Lanzettenspitze hoch ist, oder durch einen automatischen Auswurfmechanismus erfolgen.

In dem Patent EP 0 565 970 wird eine Blutlanzettenvorrichtung offenbart, bei der die Lanzette mittels einer Auswurfstange aus der Lanzettenhalterung ausgestoßen wird. Durch Betätigung eines entsprechenden Knopfes kann der Benutzer die Auswurfstange bedienen.
Des Weiteren wird in der Patentschrift US 4,442,836 ein Auswurfmechanismus beschrieben, bei dem automatisch beim erneuten Spannen der Stechhilfe die Lanzette ausgelöst wird, so dass die gebrauchte Lanzette nach jedem Einstichvorgang verworfen wird. Derartige Auswerfmechanismen erfordern einen relativ hohen zusätzlichen konstruktiven Aufwand. Des Weiteren ist eine mehrfache Benutzung eines bereits eingelegten Lanzettensystems nicht möglich, welches jedoch insbesondere im Home-Monitoring-Bereich von den Kunden häufig gewünscht ist. Ein weiterer wesentlicher Nachteil des beschriebenen Standes der Technik ist darüber hinaus, dass die Lanzettespitze nach dem Auswurf der Lanzette ungeschützt ist, so dass ein damit verbundenes Verletzungsrisiko, wie bereits beschrieben, besteht.

Um ein gefahrloses Entfernen der verbrauchten Lanzette zu erleichtern, werden im Stand der Technik weiterhin Blutentnahmesysteme beschrieben, die nach dem Auswurf der Lanzette einen Schutz vor der Lanzettespitze gewährleisten. Insbesondere für Benutzer, die im hohen Alter oder durch Folgen der Erkrankung häufig durch eine schlechte Sehfähigkeit und zitternde Hände behindert sind, wird dies als ein wesentliches Merkmal erachtet.
Um einen Schutz vor der Lanzettespitze zu realisieren, wird im Stand der Technik die Lanzette in eine Kappe der Stechhilfe integriert, so dass die Lanzette und die Gehäusekappe insgesamt eine auswechselbare disposible Einheit bilden. Derartige Konstruktionen sind in den.

Dokumenten EP 0595148 sowie US 4,990,154, US 5,454,828 und US-2004-0034318 beschrieben. Wird die Lanzette von dem Benutzer ausgeworfen, wird hierbei die Gehäusekappe über die Lanzettespitze gestülpt, so dass die Lanzette anschließend von der Kappe umgeben verworfen wird. Auch wenn bei den beschriebenen Mechanismen die Lanzettespitze nach dem Auswurf geschützt ist, so ist dennoch ein erneutes Wiedereinführen sowie ein erneuter Stechvorgang mittels einer bereits einmal ausgeworfenen Lanzette durch einen unachtsamen Benutzer möglich. Der Benutzer ist folglich darauf angewiesen, selbst zu erkennen, dass die Lanzette bereits gebraucht wurde.

Das Dokument EP 0 630 609 offenbart einen Mechanismus, der direkt das Wiedereinführen und somit ein Wiederverwenden einer einmal ausgeworfenen Lanzette verhindert. Die beschriebene Lanzettenvorrichtung beinhaltet eine Lanzette mit einem Lanzettekörper, der bei Auswurf der Lanzette aus der Stechhilfe zerbricht, so dass ein erneutes Wiedereinführen der Lanzette verhindert wird. Hierdurch wird der Benutzer gehindert eine kontaminierte Lanzette wiederzuverwenden. Nachteil des Standes der Technik ist jedoch, dass nach dem Auswurf der Lanzette die Lanzettespitze ungeschützt vorliegt.

Für einzelne Lanzetten gibt es diverse Lösungen einen Benutzer vor der benutzten Lanzettenspitze zu schützen. So werden in den Dokumenten US 5,964,731, US 7,001,364 und US 5,454,828 verschiedene Mechanismen beschrieben, wie nach Gebrauch der Lanzette ein Schutzmechanismus über die Lanzette gebracht werden kann, sodass die Lanzette nicht erneut verwendet werden kann. Diese Mechanismen sind jedoch entweder vom Benutzer selber anzuwenden, was nur bedingt einen wirkungsvollen Schutz vor Verletzung darstellt oder der Mechanismus wird automatisch nach jedem Stechvorgang durch den Stechmechanismus der Stechhilfe ausgelöst, wie in der W02008/072414, sodass die Lanzette in eingebautem Zustand nur einmal verwendet werden kann.

In der EP 1 459 683 wird ein Wiederverwendungsschutz beschrieben, der es ermöglicht nach Benutzung eines Lanzettensystems das Gehäuse des Lanzettensystems so zu verändern, dass es mit einer Stechhilfe nicht mehr regelgerecht Wechsel wirken kann. Dies wird durch eine Veränderung der Ankopplungsstelle zwischen Lanzettenmagazin und Stechhilfe erreicht. Ein erneutes Einlegen eines bereits benutzten Magazins ist hier nicht mehr möglich. Dies soll den Benutzer davor schützen, eine bereits benutzte Lanzette nochmals zu verwenden. Nachteil dieses Standes der Technik ist jedoch, dass bei dem Versuch ein bereits benutztes Magazin erneut in die Stechhilfe einzulegen die Kopplungsstelle der Stechhilfe beschädigt werden kann, wenn der Benutzer nicht sofort realisiert, dass das Magazin nicht mehr richtig mit der Ankopplungsstelle der Stechhilfe zusammenpasst.

Aufgabe der Erfindung ist es, vorteilhafter weise für den Home-Monitoring-Bereich ein leicht zu handhabendes Lanzettenmagazin zur Verfügung zu stellen, das mit einer Stechhilfe so zusammenwirken kann, dass das erneute Benutzen eines bereits ausgeworfenen Lanzettenmagazins verhindert wird, ohne dabei das Zusammenwirken des Lanzettenmagazins und der Stechhilfe zu beeinflussen, wobei gleichzeitig jederzeit beim Verwenden des Lanzettenmagazins ein Schutz vor Verletzung an der Lanzettespitze gewährleistet werden soll. Vorteilhafter weise soll eine Mehrfachverwendung einer Lanzette eines einmal eingelegten Lanzettenmagazins problemlos möglich sein.

Die Aufgabe wird gelöst durch ein Lanzettenmagazin und ein Lanzettensystem gemäß den unabhängigen Ansprüchen. Bevorzugte Ausführungsformen ergeben sich aus den abhängigen Ansprüchen.

Die Erfindung betrifft ein Lanzettenmagazin sowie ein Lanzettensystem bestehend aus einer Stechhilfe und einem Lanzettenmagazin. Weiterhin betrifft die Erfindung ein Sicherungssystem zur Sicherung der Lanzetten im Lanzettenmagazin bzw. -system.

Das Lanzettenmagazin, zur Verwendung in einer Stechhilfe, beinhaltend ein Gehäuse, mit einem distalen und einem proximalen Ende und mindestens einer Kavität für die Bevorratung von Lanzetten. In dem Lanzettenmagazin befinden sich mehrere Lanzetten mit jeweils einem Lanzettenkörper am proximalen Ende der Lanzette und einer Lanzettenspitze am distalen Ende der Lanzette, die zur Erzeugung einer Hautöffnung geeignet ist. Dabei weist das proximale Ende mindestens einer Lanzette in Richtung des proximalen Endes des Gehäuses und das distale Ende der Lanzette ist in Richtung des distalen Endes des Gehäuses ausgerichtet. Das proximale Ende des Lanzettenmagazingehäuses ist bevorzugter weise dazu ausgelegt, mit einer Stechhilfe wechselwirken zu können. Dieses Wechselwirken kann durch die Halterung des Magazins charakterisiert sein, oder durch die Ausgestaltung des proximalen Endes zum Eindringen eines Antriebselementes der Stechhilfe, z.B. in Form einer Öffnung oder Aussparung im Lanzettenmagazingehäuse. Die Lanzetten sind in unbenutztem Zustand vollständig von dem Gehäuse umgeben. Hierdurch ist zu jedem Zeitpunkt der Benutzung des Lanzettenmagazins gewährleistet, dass sich der Benutzer nicht an den Lanzetten verletzten kann.

Nur in der Entnahmepostion wird die Lanzette durch den Antriebsmechanismus der Stechhilfe in einen ausgelenkten Zustand bewegt und kann in die Haut des Benutzers stechen. Weiterhin besitzt das Lanzettenmagazin eine Verlängerungseinheit, die beweglich mit dem Gehäuse des Lanzettenmagazins verbunden ist. Sie kann in Richtung des distalen Endes des Magazingehäuses bewegt werden, sodass das Gehäuse durch die Verlängerungseinheit in Richtung des distalen Endes des Gehäuses verlängerbar ist und so ein Austreten der Lanzette aus dem Lanzettenmagazin verhindert werden kann. Hierdurch wird erreicht, dass selbst bei regelgerecht eingelegtem Lanzettenmagazin in die Stechhilfe ein Austreten der Lanzette aus dem Lanzettenmagazin selbst bei Auslösen eines Stechmechanismus nicht auftritt. Bevorzugter weise befindet sich in jeder Kavität eine Lanzette. Die Kavitäten können durch Kavitätswände voneinander abgetrennt sein. Es ist jedoch auch denkbar, dass die Kavität der Hohlraum um jede Lanzette ist und nur durch eine Nut oder Kerbe am Lanzettenmagazin voneinander getrennt sind. Als Kavität kann auch ein Hohlraum angesehen werden, in dem sich mindestens zwei Lanzetten befinden, die sich in einem definierten Abstand zueinander befinden. Nach Verlängerung des Lanzettenmagazins kann selbst bei regelrechter Kopplung des Lanzettenmagazins mit der Stechhilfe bei ausgelöstem Stechvorgang die Lanzette nicht aus dem verlängerten Lanzettenmagazingehäuse austreten.

Das Lanzettenmagazin besitzt folglich zwei Elemente, die miteinander relativ zueinander beweglich verbunden sind. Das sind zum einen das Lanzettenmagazingehäuse selbst und zum anderen die Verlängerungseinheit. Diese beiden Elemente sind so zueinander angeordnet, dass das Überführen des Lanzettemagazins von einem unverlängerten Zustand in einen verlängerten Zustand gezielt durchgeführt werden kann. Die Form und Ausgestaltung der beiden Elemente ist nicht beschränkt, solange sie beweglich so zueinander angeordnet sind, sodass in dem unverlängerten Zustand mindestens eine der im Lanzettenmagazingehäuse befindlichen Lanzetten mindestens zu einem Teil aus dem Lanzettenmagazingehäuse ausgeführt werden kann und in dem verlängerten Zustand des Lanzettenmagazins die Lanzette nicht aus dem Lanzettenmagazin austreten kann. Bevorzugter weise wird die Verlängerungseinheit beim Verlängerungsvorgang parallel zu mindestens einer Lanzette im Lanzettenmagazin bewegt. Dies ist die Lanzette, die sich in einer Entnahmeposition in dem Lanzettenmagazin bzw. Lanzettensystem befindet. Diese Entnahmeposition ist bezogen auf das Lanzettenmagazin so definiert, dass das Lanzettenmagazingehäuse an dem distalen Ende eine Öffnung aufweist, durch die die Lanzette hindurch treten kann. Bezogen auf eine Stechhilfe, in die das Lanzettenmagazin einlegbar ist, ist die Entnahmeposition so charakterisiert, dass ein Antriebselement (z.B. ein Stößel) mit einer der Lanzetten in dem Lanzettenmagazin so Wechsel wirken kann, dass diese Lanzette für einen Stechvorgang in dem Lanzettenmagazin beweglich gelagert ist. Durch die Verlängerung des Lanzettenmagazins in Richtung der Längsachse der Lanzette in der Entnahmeposition, wird gewährleistet, dass die Lanzette selbst bei Auslenkung durch eine Stechhilfe nicht aus dem Lanzettenmagazin austreten kann.

Sowohl die Verlängerungseinheit als aus das Lanzettenmagazingehäuse können aus Kunststoffmaterial gefertigt sein, aber auch andere Materialien, wie Blech sind denkbar. Bevorzugter weise weist das Lanzettenmagazingehäuse eine Trommel artige Form auf. Andere Formen, wie Quader, Zylinder oder scheibenförmige Gebilde sind ebenfalls denkbar. Das Lanzettenmagazin weist dabei mindestens eine Öffnung auf, die es ermöglicht mindestens eine Lanzette, während eines Stechvorgangs aus dem Lanzettenmagazingehäuses im unverlängerten Zustand des Lanzettenmagazins austreten zu lassen. Dabei tritt wenigstens ein Teil der Lanzette, aber mindestens die Lanzettenspitze aus dieser Öffnung aus. Mindestens um einen Teil des Lanzettenmagazingehäuses herum innerhalb oder außerhalb des Lanzettenmagazingehäuses befindet sich die Verlängerungseinheit, die mindestens im Bereich der Öffnung beweglich mit dem Lanzettenmagazingehäuse verbunden ist. Diese Verbindung kann über Führungsnuten stattfinden. Bevorzugter weise befindet sich die Verlängerungseinheit außerhalb des Lanzettenmagazingehäuses und ist mindestens in zwei Dimensionen der Form des Lanzettenmagazingehäuses angepasst. Dabei weist die Verlängerungseinheit bevorzugter weise einen Formschluss mit dem Lanzettenmagazingehäuse auf. Darüber hinaus wird die Verlängerungseinheit in der Bewegungsrichtung in ihrer Bewegung in beide Richtungen begrenzt, sodass ein unbeabsichtigtes Abrutschen der Verlängerungseinheit vom Lanzettenmagazingehäuse verhindert wird. Diese Begrenzung kann zum einen durch die Führungselemente wie Nut und Kerbe erreicht werden, die in ihrer Form und Länge so aufeinander abgestimmt werden, dass die Bewegung entlang einer Achse begrenzt wird. Andererseits kann die Verlängerungseinheit an einem Ende eine Verjüngung gegenüber der Dimension des Lanzettenmagazingehäuses vorsehen, sodass eine Begrenzung der Bewegung durch die Verjüngung erreicht wird. Da das Ziel ist, die Verlängerungseinheit gezielt so zu dem Lanzettenmagazingehäuse auszurichten bzw. bewegen zu können, dass die Lanzetten selbst bei Auslenkung aus dem Lanzettenmagazingehäuse im verlängerten Zustand nicht aus dem Lanzettenmagazin austreten, sollte die Verlängerungseinheit mindestens in Richtung der Öffnung des Lanzettenmagazingehäuses bewegt werden können, um die Lanzette in der Entnahmeposition bei gewolltem oder ungewolltem Stechvorgang nicht aus dem Lanzettenmagazin austreten zu lassen. Die Verlängerungseinheit weist in ihrer Ausdehnung in der Richtung, in der die Verlängerung stattfindet, bevorzugter weise eine Länge auf, die mindestens der Länge des Teils der Lanzette entspricht, der beim regelrechten Stechvorgang aus dem Lanzettenmagazingehäuse heraustritt. Die Länge der Verlängerungseinheit kann jedoch auch darüber hinaus gehen. Erfindungsgemäß sollte jedoch mindestens die Länge des austretenden Lanzettenteils beim normalen Stechvorgang durch die Verlängerungseinheit im verlängerten Zustand überdeckt werden.

Durch die Bewegung der Verlängerungseinheit wird das Lanzettenmagazin folglich unbrauchbar gemacht und gleichzeitig wird weiterhin ein Schutz für den Benutzer gegen ein unbeabsichtigtes Stechen gewährleistet, da die Lanzetten vollständig von dem Lanzettenmagazin umgeben sind. Durch die Verlängerung des Lanzettenmagazins in distaler Richtung ist gewährleistet, dass die proximale Seite des Magazins, die mit einer Stechhilfe wechselwirken kann nicht beeinträchtigt wird. Hierdurch wird ermöglicht, dass zwar eine Wiederverwendung des Lanzettenmagazins nach Verlängerung des Magazins verhindert wird, ohne dass das Einlegen des Magazins beeinträchtigt wird. Dies hat den Vorteil, dass bei versehentlichem Einlegen eines verbrauchten Lanzettenmagazins in die Stechhilfe nicht die Andockstelle zwischen Lanzettenmagazin und Stechhilfe beeinträchtigt wird. Es kann dadurch verhindert werden, dass die Stechhilfe, die ja viele Jahre für den Benutzer zur Verfügung stehen soll, durch ein verändertes Lanzettenmagazin manipuliert wird, dass sie unbrauchbar gemacht wird, wie dies in der EP 1 459 683 der Fall ist, bei der eine Veränderung des Lanzettenmagazins am proximalen Ende des Magazins stattfindet, um ein Wiederverwenden in Form eines Wiedereinlegeschutzes zu bewerkstelligen.

Das Bewegen der Verlängerungseinheit kann automatisch bei Entfernen des Magazins aus der Stechhilfe eintreten. Dies kann beispielsweise durch einen automatischen Auswurfmechanismus der Stechhilfe erfolgen, der durch den Benutzer bei verbrauchtem Magazin betätigt werden kann. Die Verlängerungseinheit kann jedoch auch per Hand durch den Benutzer an dem Lanzettenmagazin entlang bewegt werden, wenn er händisch das Magazin aus der Stechhilfe herausnimmt. Dabei sind das Lanzettenmagazingehäuse und die Verlängerungseinheit im nicht verlängerten Zustand so miteinander verbunden, dass die Verlängerungseinheit nur bei übersteigen einer bestimmten Krafteinwirkung in den verlängerten Zustand überführbar ist. Dies kann zum Beispiel dadurch erreicht werden, dass Teile des Lanzettenmagazingehäuses und der Verlängerungseinheit so dicht aneinander gelagert sind, dass erst eine bestimmte Reibungskraft überwunden werden muss, bis die Verlängerungseinheit bewegbar ist. Diese Sicherung des unverlängerten Zustandes kann alternativ durch Elemente an Lanzettenmagazingehäuse und Verlängerungseinheit erreicht werden, wie beispielsweise Kerben oder Vorsprünge die gegeneinander reiben oder ineinander verhaken bzw. verrasten. Eine ähnliche Sicherung findet auch im verlängerten Zustand des Lanzettenmagazins statt.

In einer bevorzugten Ausführungsform ist das Magazin dabei so in die Stechhilfe einlegbar, dass der Benutzer das Magazin auch herausnehmen kann, ohne den Verlängerungsmechanismus zu betätigen. Hierdurch ist es dem Benutzer möglich auch ein Lanzettenmagazin aus der Stechhilfe zu entnehmen, dass noch nicht vollständig verbraucht ist, ohne dass es durch die Verlängerung unbrauchbar gemacht wird. Dies kann entweder dadurch erreicht werden, dass die Verlängerungseinheit innerhalb des Lanzettenmagazingehäuses sich erstreckt und nur am distalen Ende des Lanzettenmagazingehäuses aus diesem hervorsteht und ergriffen werden kann, um den Verlängerungsprozess vorzunehmen. In einer alternativen Ausführungsform kann das Lanzettenmagazingehäuse so lang gestreckt sein, dass es in eingebautem Zustand innerhalb einer Stechhilfe greifbar für den Benutzer gelagert ist, sodass der Benutzer die Möglichkeit hat nicht an der Verlängerungseinheit das Lanzettenmagazin aus der Stechhilfe zu entnehmen und dabei den Verlängerungsmechanismus auszulösen, sondern am Lanzettenmagazingehäuse selbst anzufassen und das Lanzettenmagazin zu entnehmen, ohne dass der Verlängerungsmechanismus benutzt wird.

Des Weiteren ist es möglich, die Lanzette, die sich in der Entnahmeposition in dem Lanzettensystem befindet, mehrmals zu verwenden. Der Benutzer kann folglich frei wählen, wie oft er jede Lanzette benutzt, bevor er eine neue Lanzette in die Entnahmeposition bewegt, z. B. durch weitertakten des Magazins in der Stechhilfe. Der Verlängerungsvorgang kann jedoch auch am Lanzettenmagazin durchgeführt werden, ohne dass das Magazin in die Stechhilfe eingebaut ist.

Das Lanzettenmagazin ist auch in verlängertem Zustand jederzeit in die Stechhilfe einlegbar. Dabei kann das Lanzettenmagazingehäuse mit seinem proximalen Ende in die Stechhilfe eingeführt werden, sodass eine regelgerechte Wechselwirkung zwischen Stechhilfe und Magazin stattfinden kann. Der Stechvorgang als solcher, bei der die Antriebseinheit mit der Lanzette wechselwirkt und diese innerhalb der Stechhilfe bewegt, kann dabei weiterhin ausgeführt werden, ohne dass jedoch die Lanzette aus dem Lanzettenmagazin austritt.

Die Verlängerungseinheit wechselwirkt nach Bewegen in distaler Richtung relativ zum Gehäuse des Lanzettenmagazins, also im verlängerten Zustand, mit dem Gehäuse, sodass sie manuell nicht wieder in proximale Richtung des Gehäuses bewegbar ist. Dies wird bevorzugter weise durch ein Einrasten zwischen Verlängerungseinheit und Lanzettenmagazingehäuse bewerkstelligt. Das Zusammenwirken von zwei Kerben oder von Nut und Kerbe an jeweils der Verlängerungseinheit und dem Lanzettenmagazingehäuse sind Beispiele für solch eine Verrastung der beiden Elemente. Dadurch kann verhindert werden, dass die Verlängerungseinheit sich von alleine wieder in ihre ursprüngliche Länge im unverlängerten Zustand bewegt.

Die Erfindung betrifft weiterhin ein Lanzettensystem zur Ausführung eines Stechvorgangs in einen Körper, beinhaltend eine Stechhilfe mit einem Gehäuse und einem Antriebsmechanismus für eine Lanzette sowie einer Aufnahmeöffnung für ein Lanzettenmagazin, ein Lanzettenmagazin mit einem Gehäuse, das ein distales und ein proximales Ende aufweist, beinhaltend mindestens eine Lanzette, wobei die Lanzette an ihrem distalen Ende eine Spitze aufweist und mit dem distalen Ende in Richtung distalem Ende des Lanzettenmagazins weist, eine Verlängerungseinheit, die verschiebbar mit dem Gehäuse des Lanzettenmagazins verbunden ist.

Das Lanzettenmagazin ist dabei durch die Verlängerungseinheit in Richtung des distalen Endes des Gehäuses verlängerbar, sodass ein Austreten der Lanzetten aus dem Lanzettenmagazin bei Wechselwirkung mit der Stechhilfe verhindert wird. Das Lanzettenmagazin wird dabei mit seinem proximalen Ende in die Stechhilfe eingeführt, da das Magazin bevorzugter weise an einem proximalen Ende dazu ausgelegt ist, mit der Stechhilfe so wechselwirken kann, dass es gehalten wird.

Ein wichtiger Aspekt der Erfindung stellt ein von der Antriebseinheit abnehmbares Lanzettenmagazin dar, in dem sich mindestens eine Lanzette befindet, wobei das Lanzettenmagazin als disposible Einheit vorgesehen ist. Hierbei umfasst der Begriff Lanzette sowohl eine klingenförmige, flach ausgeprägte Stecheinheit sowie andere denkbare Ausführungsformen. Prinzipiell können im Rahmen der Erfindung Lanzetten eingesetzt werden, wie sie grundsätzlich im Stand der Technik hinlänglich bekannt sind und in einem Lanzettensystem Anwendung finden. Im Stand der Technik wird dabei häufig die Kombination einer Lanzette mit einem Grundkörper, der an die Stechhilfe ankoppeln kann, als Lanzette bezeichnet. Solche Lanzetten weisen oftmals einen Grundkörper aus Kunststoff auf, in dem eine Metalllanzette angeordnet ist. Erfindungsgemäß ist es möglich, eine derartige Lanzette in das erfindungsgemäße Lanzettenmagazin bzw. -system zu integrieren. Es ist z. B. denkbar, dass der erfindungsgemäße Lanzettekörper einen Grundkörper, wie er im Stand der Technik bei Lanzetten eingesetzt wird, enthält, wobei durch die Integration des Grundkörpers die erfindungsgemäße Funktionalität des Systems gewahrt bleibt. Der Lanzettekörper ist dann entsprechend der beschriebenen Ausgestaltung mindestens zweiteilig ausgebildet. Die Lanzette und der Grundkörper sind innerhalb des Magazins, dann beweglich führbar. Vorzugsweise befinden sich die Lanzetten innerhalb des Lanzettenmagazins in voneinander separierten Kavitäten, um eine Kontamination ungebrauchter Lanzetten durch bereits verwendete Lanzetten bei einer Remagazinierung zu verhindern.

Zum Ausführen eines Stechvorgangs sind Bereiche des Lanzettekörpers vorteilhafter weise analog zu der in DE 10053974 bereits dargestellten Systems ausgestaltet, so dass die einzelnen Lanzetten des Systems mit der Antriebseinheit der Stechhilfe aktiv koppeln können. Ausführungsformen, die ebenfalls zum Antreiben von Lanzetten innerhalb eines Magazins einer Stechhilfe dienen können, sind z. B. in den Dokumenten DE 10053974, US 4,990,154 und US 5,074,872 beschrieben. Die nebeneinander angeordneten Kammern, in denen sich die Lanzetten einzeln befinden, werden zum Ausführen eines Stechvorgangs nacheinander relativ zur Antriebseinheit so positioniert, dass jeweils eine einzelne Lanzette an den Stößel der Antriebseinheit angekoppelt werden kann. Besonders vorteilhaft erweisen sich auch hier Magazine in Form einer Trommel mit parallel zur Längsachse der Trommel angeordneten Kammern, in denen sich die Lanzetten befinden. Bevorzugter weise besitzen die Lanzetten an ihrem Lanzettenkörper zusätzlich Lanzettenkörperarme, die mit der Antriebseinheit der Stechhilfe so wechselwirken können, dass eine formschlüssige Verbindung zwischen Antriebseinheit (z.B. in Form eines Stößels) und der Lanzette stattfinden kann, sodass eine geführte Bewegung der Lanzette während des Stechvorgangs erfolgen kann.

Das Lanzettensystem kann eine Stechhilfe mit einer aufsetzbaren Kappe beinhalten, die abnehmbar mit dem Gehäuse der Stechhilfe verbindbar ist. Nach dem Verlängerungsvorgang des Lanzettenmagazin durch die Bewegung des Verlängerungseinheit relativ zum Lanzettenmagazingehäuse, wobei die Verlängerungseinheit in Richtung des distalen Endes des Gehäuses des Lanzettenmagazins bewegt wird, ist die Kappe nicht mehr regelgerecht mit dem Gehäuse der Stechhilfe verbindbar. Dies ist ein weiteres Indiz für den Benutzer der Stechhilfe, dass das Lanzettenmagazin, das er in der Stechhilfe verwenden will, bereits benutzt ist.

Des Weiteren wird ein Verfahren zur Vermeidung einer Wiederverwendung eines Lanzettenmagazins beschrieben, wobei das unbenutzte Lanzettenmagazins mit mindestens einer Lanzette in eine Stechhilfe eingelegt wird, wobei das Lanzettenmagazin aus der Stechhilfe wieder entnehmbar ist und wobei eine bewegbare Verlängerungseinheit am Lanzettenmagazin in Richtung distalem Ende des Lanzettenmagazins bewegt wird.

Die Lanzetten in dem Lanzettenmagazin können durch Sicherungsmittel gegen eine Bewegung relativ zum Lanzettenmagazingehäuse, verursacht durch unbeabsichtigte Krafteinwirkung, gesichert sein. Solch eine unbeabsichtigte Krafteinwirkung kann beispielsweise ein Herunterfallen des Lanzettenmagazins allein oder in Kombination mit einer Stechhilfe sein in die das Lanzettenmagazin eingebaut ist.

Dieses Sicherungsmittel kann beispielsweise eine Kunststoffummantelung um die Lanzettenspitze sein. Diese kann bei ausreichendem Krafteintrag auf die Lanzette durch eine Stechhilfe durchstechbar sein. Dieser ausreichende Krafteintrag ist beispielsweise bei einem normal durchgeführten Stechvorgang gegeben, bei dem die Antriebseinheit der Stechhilfe z.B. in Form eines Stößels auf die Lanzette bewegt wird und die Lanzette durch diesen Kraftstoß in einer geführten Bewegung mit ihrer Lanzettenspitze aus dem Gehäuse der Stechhilfe getrieben wird. Hingegen reicht der Krafteintrag beim Sturz eines Lanzettenmagazins aus einer Höhe von beispielsweise 2m nicht aus, um das Kunststoffmaterial zu durchstechen. Die Schwelle bei der der Krafteintrag ausreicht, um den Kunststoff zu durchstechen kann durch die Wahl der Zusammensetzung des Kunststoffs beeinflusst werden. Wie ein solcher Kunststoff aufgebaut sein kann und wie er mit der Lanzette so verbunden werden kann, dass er sowohl die Funktion des Sterilschutzes als auch des Sicherungsmittels der Lanzette bei einem Fall übernehmen kann, ist in der US 2003-0153939 beschrieben.

Zusätzlich kann das Lanzettensystem eine Lanzettenhalterung zur Fixierung von Lanzetten in einem Lanzettenmagazin aufweisen, wobei die Stechhilfe eine Aussparung zur Aufnahme eines Lanzettenmagazins aufweist, und die Stechhilfe eine Entnahmeposition aufweist, in der ein Antriebselement mit einer der Lanzetten in dem Lanzettenmagazin so wechselwirken kann, dass diese Lanzette für einen Stechvorgang in dem Lanzettenmagazin beweglich gelagert ist. Die Stechhilfe weist dabei weiterhin ein Sicherungselement auf, das eine Bewegung der Lanzetten, die nicht in der Entnahmeposition positioniert sind, verhindert. Bevorzugter weise ist dieses Sicherungselement in Form eines Dorns ausgebildet ist, der bei Einlegen des Lanzettenmagazins in die Stechhilfe mindestens zu einem Teil in das Lanzettenmagazin eindringt. Bei dem Einschieben des Lanzettenmagazins in die Stechhilfe wird dieser Dorn so zu dem Lanzettenmagazin positioniert, dass die Lanzettenkörperarme der Lanzetten, die nicht in der Entnahmeposition sind, in einer Ausnehmung des Lanzettenmagazingehäuses fixiert werden. Die Lanzette die sich in der Entnahmeposition befindet, wird beispielsweise durch den Stößel der Antriebseinheit der Stechhilfe gesichert, in dem die Lanzettenkörperarme und der Stößel einen Formschluss bilden. Der Dorn weist bevorzugter weise eine längliche Stiftform auf, ist also vorteilhafter weise rund ausgeformt. Durch die runde Form bildet er wenig Widerstand beim Einführen des Lanzettenmagazins in die Stechhilfe. Die gezielte Fixierung nur der Lanzetten, die nicht in der Entnahmeposition sind wird beispielsweise dadurch bewerkstelligt, dass der Dorn zumindest zu einem Teil asymmetrisch in das Lanzettenmagazin eingeführt wird. Alternativ kann der Dorn eine Aussparung für das Antriebselement der Stechhilfe aufweisen, welches während des Stechvorgangs parallel zu dem Dorn im Gehäuse der Stechhilfe bewegt wird. Durch diese Fixierung sind die Lanzetten in dem Lanzettensystem bzw. in dem Lanzettenmagazin vor unerwünschter Verschiebung geschützt. Eine unerwünschte Verschiebung der Lanzetten wäre beispielsweise das Herunterfallen der Stechhilfe mitsamt dem Lanzettenmagazin. Aber auch Stürze aus höherer Distanz, z.B. aus bis zu 3m Höhe können durch diesen Mechanismus abgefangen werden, ohne dass ein Risiko besteht, dass eine Lanzette unbeabsichtigt aus der Stechhilfe oder dem Lanzettenmagazin austritt. Dadurch ist eine Verletzungsgefahr durch falsches Bedienen der Stechhilfe weitestgehend ausgeschlossen.

Eine wichtige Anforderung an das Lanzettensystem liegt in der Sterilität der Lanzettespitze, die zur Erzeugung einer Wunde in einem dafür vorgesehenen Körperteil vorgesehen ist. Die Sterilität der Lanzettespitze muss dabei über einen langen Zeitraum gewährleistet sein, der sich von der Produktion des Lanzettensystems bis zu seinem Gebrauch erstreckt. Bei der Herstellung des Lanzettensystems kann eine Sterilität, wie im Stand der Technik üblich, z. B. durch Gamma-Strahlung erzielt werden. Zum Aufrechterhalten der Sterilbedingung kann das Lanzettensystem in eine Umverpackung, beispielsweise einen Polyethylenbeutel, eingeschweißt werden. In einer anderen Ausführungsform kann beispielsweise die Öffnung des Lanzettensystems zum Austritt der Lanzettespitze aus dem Schutzbereich des Lanzettekörpers durch Siegelfolie verschlossen werden. Diese können vorzugsweise abziehbare Siegelfolien sein, die der Benutzer vor dem Gebrauch des Lanzettensystems entfernt. Es können jedoch auch dünne Folien verwendet werden, die erst bei der Benutzung der Lanzette von der Lanzettespitze durchstoßen werden, so dass dem Benutzer zusätzliche Handhabungsschritte erspart bleiben. Solche Folien können bereits integral im Herstellungsprozess des Lanzettensystems, in der Regel mittels eines Spritzgussprozesses, eingesetzt werden.

Des Weiteren wird im Stand der Technik in der Anmeldung WO 01/66010 ein Sterilschutz durch ein Elastomer beschrieben, das die Lanzettespitze einschließt und sie somit vor Kontamination schützt. Dieser Sterilschutz kann entweder während des Stechvorgangs durchstochen werden oder vor Gebrauch von dem Benutzer entfernt werden.

In einer vorteilhaften Ausführungsform kann der Schutzbereich des Lanzettekörpers einen Sterilschutz beinhalten und/oder durch diese im Wesentlichen gebildet werden. Hierbei dient z. B. das Elastomer des Sterilschutzes selbst als Schutzbereich des Lanzettekörpers, in dem die Lanzettespitze relativ zu dem Elastomer beweglich geführt werden kann. Dabei gibt der Sterilschutz reversibel die Lanzettespitze frei bzw. umschließ sie wieder, wie es zum Beispiel im Falle eines Elastomerenschutzes (US 2003-0153939) gegeben ist, bei dem das Elastomer während des Stechvorgangs zunächst durchstochen und anschließend die Lanzettespitze wieder in das Elastomer zurückgezogen wird. Im aufgeführten Beispiel ändert folglich die Lanzettespitze ihre Position relativ zum Sterilschutz während des Stechvorgangs, wobei die Lanzettespitze nach dem Stechvorgang in ihrer Ruheposition vom Sterilschutz geschützt wird. Prinzipiell sind viele mögliche Ausführungsformen eines Sterilschutzes denkbar, so dass das erfindungsgemäße System auf keine spezielle Ausführungsform eines Sterilschutzes beschränkt ist.

### Beschreibung der Figuren

- Fig. 1a:: Seitenansicht eines Lanzettenmagazins mit Verlängerungseinheit in unverlängerten Zustand.
- Fig. 1b:: Seitenansicht eines Lanzettenmagazins mit Verlängerungseinheit in verlängertem Zustand.
- Fig. 2a:: Ansicht des Lanzettenmagazins aus Fig. 1a im Längsschnitt entlang einer Schnittlinie B-B.
- Fig. 2b:: Ansicht des verlängerten Lanzettenmagazins aus Fig. 1b im Längsschnitt entlang der Schnittlinie B-B.
- Fig. 3a:: Ansicht des Lanzettenmagazins aus Fig. 2a entlang einer Schnittlinie A-A.
- Fig. 3b:: Ansicht des Lanzettenmagazins aus Fig. 2b im Längsschnitt entlang der Schnittlinie A-A aus Fig. 2b.
- Fig. 4a:: Ansicht eines Lanzettenmagazins, eingebaut in eine Stechhilfe, die eine abnehmbare Schutzkappe aufweist, in nicht verlängertem Zustand im Längsschnitt entlang der Schnittlinie A-A.
- Fig. 4b:: Längsschnitt entlang Schnittlinie A-A eines Lanzettenmagazins in verlängertem Zustand, eingebaut in einer Stechhilfe mit abnehmbarer Schutzkappe, wobei die Schutzkappe keine Verrastung mit der Stechhilfe mehr aufweist.
- Fig. 5a:: Seitenansicht einer Stechhilfe mit Fingerkappe, die korrekt eingerastet ist, da das eingelegte Lanzettenmagazin in nicht verlängertem Zustand ist.
- Fig. 5b:: Längssschnitt durch eine Stechhilfe aus Fig. 5a mit Fingerkappe und eingelegtem Lanzettenmagazin in nicht verlängertem Zustand entlang der Schnittlinie A-A.
- Fig. 5c:: Längsschnitt einer Stechhilfe mit eingelegtem Lanzettenmagazin nach Verlängerung und aufgesteckter Fingerkappe, die nicht korrekt mit der Stechhilfe verrastet werden kann (Schnittlinie A-A).
- Fig. 5d:: Seitenansicht einer Stechhilfe mit Fingerkappe, die nicht mit der Stechhilfe verrasten kann, da das Lanzettenmagazin in verlängertem Zustand ist.

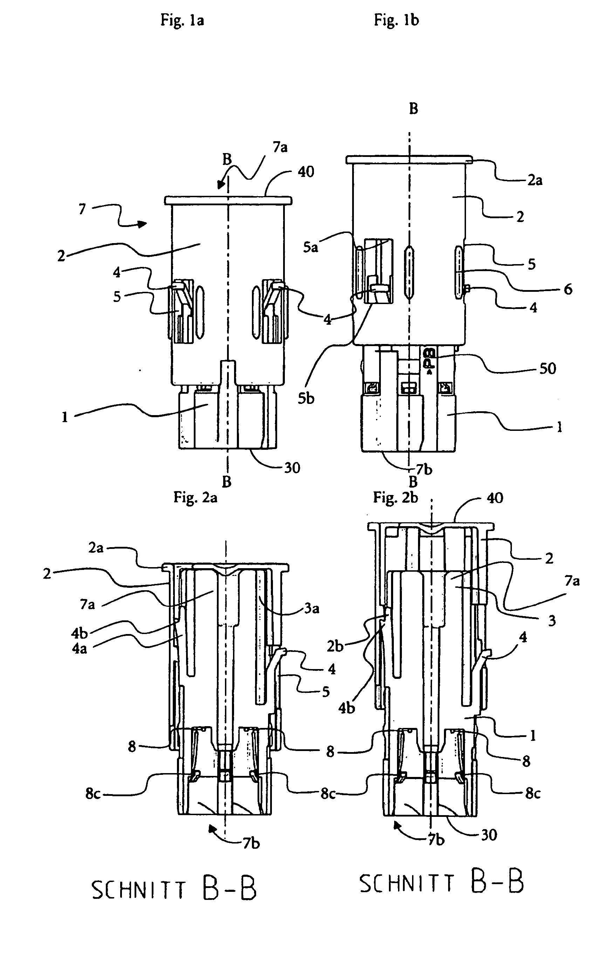

Das in den Figuren 1 bis 5 gezeigte Lanzettenmagazin (7) weist eine zylindrische Form auf, was jedoch nicht bedeutet, dass das Magazin nicht auch jede andere Form aufweisen kann, wie beispielsweise eine Kastenform oder ein Quarder. Die hier gezeigte zylindrische Form des Lanzettenmagazins (7) ermöglicht eine Magazinierung der Lanzetten in Längsrichtung des zylindrischen Gehäuses (1). Auf diese Weise liegen die Lanzetten parallel zueinander in dem Gehäuse (1).

Figur 1a zeigt ein Lanzettenmagazin (7) mit einem Gehäuse (1), das die bevorrateten Lanzetten (hier nicht gezeigt) vollständig umschließt. Das Lanzettenmagazin (7) weist an einem Ende (dem proximalen Ende) eine Öffnung (30) auf, durch die eine Stechhilfe (hier nicht gezeigt) mit den Lanzetten im Inneren des Lanzettenmagazins (7) wechselwirken kann. Auf der gegenüberliegenden Seite (dem distalen Ende) des Lanzettenmagazins (7) befindet sich ebenfalls mindestens eine Öffnung (40), die es ermöglicht, dass eine Lanzette beim Stechvorgang aus dem Magazin (7) austreten kann. Das Lanzettenmagazin (7) weist darüber hinaus eine Verlängerungseinheit (2) auf, die beweglich mit dem Gehäuse (1) verbunden ist. Bevorzugter weise ist diese Verlängerungseinheit (2) ebenfalls zylindrisch ausgestaltet und verläuft in ihrer Längsausrichtung parallel zu dem Gehäuse des Lanzettenmagazins (2), wobei mindestens ein Teil dieser Verlängerungseinheit mit dem Gehäuse (1) beweglich wechselwirkt. In der speziellen Ausführungsform der Figuren 1 bzw. 2 überspannt die Verlängerungseinheit (2) einen Teil des Lanzettenmagazingehäuses (1) in ihrer Längsausdehnung und ragt am distalen Ende des Lanzettenmagazingehäuses über das Lanzettenmagazingehäuse (1) hinaus. An diesem distalen Ende der Verlängerungseinheit (2) weist diese eine Griffkante (2a) auf, wobei dies eine Verdickung des Querschnitts der Verlängerungseinheit (2) darstellt. Darüber hinaus weist die Verlängerungseinheit (2) mindestens einen seitlichen Schlitz (5) sowie mindestens eine Führung (6) an ihrer Längsseite auf. Dabei dient der Seitenschlitz (5) dazu, um einen Verrastungsarm (4) der an dem Lanzettenmagazingehäuse (1) angebracht ist, aufzunehmen. Dabei bewegt sich der Verrastungsarm (4) während des Verlängerungsprozesses des Lanzettenmagazingehäuses (1), durch die Verlängerungseinheit (2), von dem distalen Ende des Seitenschlitzes (5a) zum proximalen Ende des Seitenschlitzes (5b), sodass der Verrastungsarm (4) die Verlängerungseinheit (2) daran hindert, weiter über das Lanzettenmagazingehäuse (1) hinausbewegt zu werden. Es stellt folglich eine Begrenzung des Verlängerungsweges dar, sodass die Verlängerungseinheit (2) nicht komplett vom Lanzettenmagazingehäuse (1) abgezogen werden kann. In Figur 1a ist folglich der Verrastungsarm (4) am oberen (distalen) Ende des Seitenschlitzes (5a) zu erkennen, während er in Figur 1b nach Verlängerung des Lanzettenmagazins (7) am proximalen Ende des Seitenschlitzes (5b) zu finden ist. Darüber hinaus weist das verlängerte Lanzettenmagazin (1) aus Figur 1b eine optische Markierung (50) in Form von Buchstaben auf. Diese optische Markierung (50) kann jedoch auch in Form einer farblichen Markierung bevorzugter weise am Lanzettenmagazingehäuse (1) ausgestaltet sein.

In Figur 2a ist ein Querschnitt des Lanzettenmagazins (7) aus Figur 1a in der Schnittrichtung B-B gezeigt. In dieser Ansicht ist die Anordnung des Verrastungsarms (4) innerhalb des Lanzettenmagazingehäuses (1) zu erkennen, wobei das eine Ende des Verrastungsarms aus dem Seitenschlitz (5) der Verlängerungseinheit (2) herausragt. An einer anderen Stelle des Lanzettenmagazingehäuses (1) ist ein weiterer Verrastungsarm (4a) angebracht, der innerhalb der Verlängerungseinheit (2) angeordnet ist. Dieser Verrastungsarm (4a) weist eine Kerbe (4b) auf, die mit einer Kerbe (2b) der Verlängerungseinheit (2) während eines Verlängerungsvorgangs des Lanzettenmagazingehäuses (1) so wechselwirken kann, dass wie in Figur 2b dargestellt, eine Verkürzung des Lanzettenmagazingehäuses (1) nicht mehr möglich ist. Dies wird in diesem Fall durch Anschrägen der Kerben (4b) und (2b) in entgegengesetzte Richtungen erreicht, sodass eine formschlüssige Verrastung dieser beiden Elemente stattfindet. Es sind auch andere Wechselwirkungen zwischen Magazingehäuse (1) und Verlängerungseinheit (2) möglich, die nach der Verlängerungsphase eine Rückbewegung unterbinden. So wäre es auch denkbar, dass die Kerbe (4b) in eine Nut der Verlängerungseinheit (2) eingreift und dort verrastet. Während des Verlängerungsvorgangs wird der innere Verrastungsarm (4a) von der Kerbe (2b) in das Lanzettenmagazininnere gedrückt, bis die Kerbe (5b) an der Kerbe (4b) vorbeigeglitten ist. Durch das vorbei Gleiten der beiden Kerben aneinander wird ein gewisser Reibungswiderstand aufgebaut, der während des Verlängerungsprozesses überwunden werden muss. Dadurch wird gewährleistet, dass das Lanzettenmagazin (7) nicht von alleine oder durch geringe Krafteinwirkung in seiner Längsausrichtung durch Verschieben der Verlängerungseinheit (2) gegen das Lanzettenmagazingehäuses (1) verlängert wird. Obwohl in dieser Ansicht von Figur 2a und 2b die eingelegten Lanzetten (8) nicht vollständig zu erkennen sind, da eine Kavitätsbegrenzung (3a) diese bedeckt, sind immerhin die Lanzettenkörperarme (8c) von zwei Lanzetten (8) unterhalb der Kavitätsbegrenzungen (3a) zu erkennen.

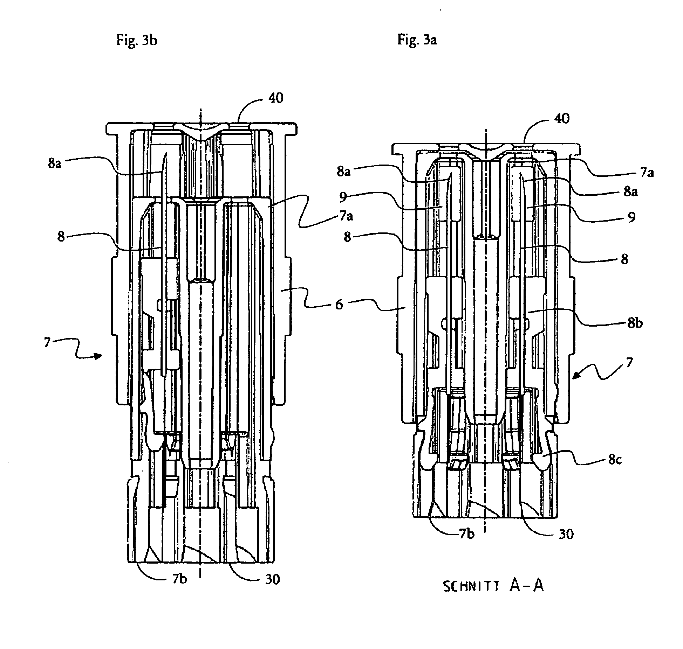

In Figur 3a sind die magazinierten Lanzetten (8) mit den Lanzettenspitzen (8a) sowie den Lanzettenkörpern (8b) und den Lanzettenkörperarmen (8c) in der Schnittebene A-A zu der Figur 2a zu erkennen. Darüber hinaus ist in dieser Schnittansicht ein Sterilschutz (9) zu erkennen, der mindestens die Lanzettenspitze (8a) umgibt. Dieser Sterilschutz (9) dient zum steril halten der Lanzettenspitze (8a) während der Aufbewahrungsphase und kann während des Antriebsvorgangs der Lanzette (8) von der Lanzettenöffnung (40) aufgehalten werden, wobei ein Stößel der Stechhilfe mit dem Lanzettenkörperarm (8c) wechselwirken kann und die Lanzette (8) in Richtung der Öffnung (40) des Lanzettenmagazins bewegt, sodass die Lanzettenspitze (8a) für den Stechvorgang freigelegt wird. Dieser Sterilschutz (9) kann zusätzlich die Aufgabe der Sicherung der Lanzetten (8) in dem Lanzettenmagazin (7) übernehmen, während das Lanzettenmagazin (7) in Benutzung ist, also in eine Stechhilfe eingebracht ist, aber auch außerhalb einer Stechhilfe. Dabei sichert der Sterilschutz (9) die Lanzetten (8) gegen ein unbeabsichtigtes Heraustreten aus den Öffnungen (40), bei geringer Krafteinwirkung, z. B. durch das Herunterfallen des Lanzettenmagazins (7).

In Figur 3b ist das Lanzettenmagazin (7) nach dem Verlängerungsprozess zu sehen, wobei selbst die in Maximalauslenkung befindliche Lanzette (8) mit ihrer Lanzettenspitze (8a) nicht aus dem Lanzettenmagazin (7) herausragt. Die Maximalauslenkung wird zum Beispiel beim regelrechten Antrieb der Lanzette (8) in einer Stechhilfe (hier nicht gezeigt) erreicht, wie sie bei einem üblichen Stechvorgang ausgeführt wird, um die Haut eines Patienten zur Blutgewinnung zu stechen.

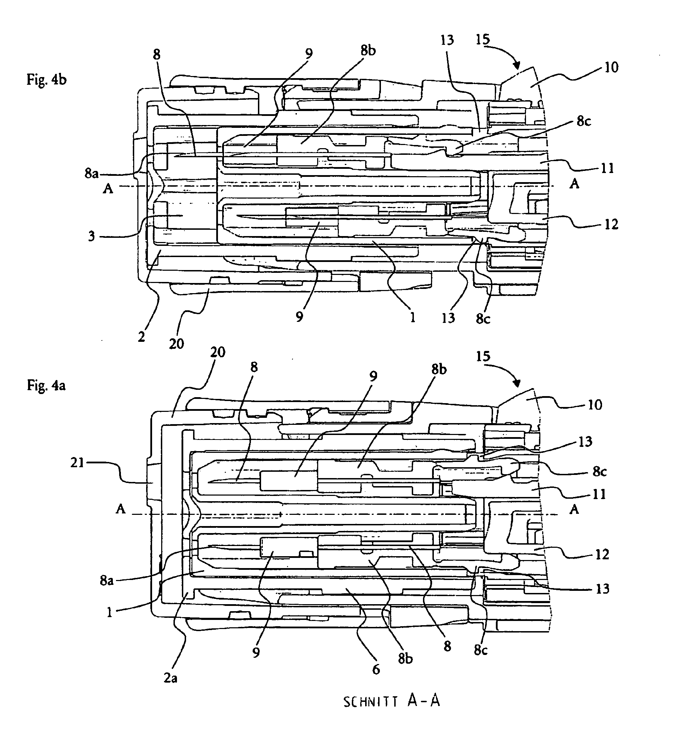

Dieser Zustand ist in Fig. 4b gezeigt, in dem das Lanzettenmagazin (7) eingebaut in eine Stechhilfe (15) vorliegt, nachdem das Lanzettenmagazingehäuse (1) verlängert wurde. Hierbei ist das Ankoppeln des Lanzettenmagazins (7) an die Stechhilfe (15) zwar regelgerecht möglich, was bedeutet, dass die Verrastungsmöglichkeiten zwischen Lanzettenmagazin (7) und Stechhilfe (15) nicht beeinträchtigt sind. Auch das Ankoppeln des Stößels (11) ist weiterhin möglich, sodass die Lanzette (8) innerhalb des Lanzettengehäuses (1) zum distalen Ende des Lanzettenmagazins (7a) bewegt wird, aber nicht aus dem durch die Verlängerungseinheit (2) verlängerten Lanzettenmagazins (7) austreten kann und folglich kein Stechvorgang ausgeübt werden kann. In dieser Ausführungsform ist jedoch das Aufsetzen der Fingerkappe (20) nicht mehr so möglich, dass das Gehäuse der Stechhilfe (10) mit der Fingerkappe (20) verrasten kann.

Dies ist anders in dem nicht verlängerten Zustand des Lanzettenmagazins (7), das regelgerecht in eine Stechhilfe (15) eingebaut ist, wie in Fig. 4a gezeigt. Hier ist ebenfalls das Lanzettenmagazin (7) über einen Dorn (12) zentriert in die Stechhilfe (15) eingeführt, sodass der Stößel (11) bei einem Stechvorgang mit der Lanzette (8) über den Lanzettenkörperarm (8c) so Wechsel wirken kann, dass die Lanzette (8) in dem Magazin geführt bewegt wird und die Lanzettenspitze (8a) aus der Öffnung (21) der Fingerkappe (20) aus der Stechhilfe (15) herausbewegt wird. Dabei ist der Dorn (12) so asymmetrisch in das Lanzettenmagazin (7) eingeführt, dass er zwar die Bewegung von Stößel (11) mit der Lanzette (8), die sich gerade vor der Öffnung der Fingerkappe (21) befindet, nicht behindert, aber aller anderen Lanzette (8) im Magazin vor einer ungewollten Bewegung schützt. Dieser Bewegungsschutz wird dadurch erreicht, dass die Lanzettenkörperarme (8c) der anderen Lanzetten (8) sich in einer Nut (13) des Lanzettenmagazins (1) (oder alternativ zwischen Lanzettenmagazin (1) und Stechhilfe (15)) befindet. Der Dorn überragt einen Teil der Lanzettenkörperarme (8c) soweit, dass selbst bei einer Krafteinwirkung auf die Stechhilfe (15) oder auf das Lanzettenmagazin (7) die Lanzetten (8) nicht aus ihrer Position verrutschen können, da der Lanzettenkörperarm (8a) in der Nut (13) zwischen Dorn (12) und Lanzettenmagazingehäuse (1) eingeklemmt sind.

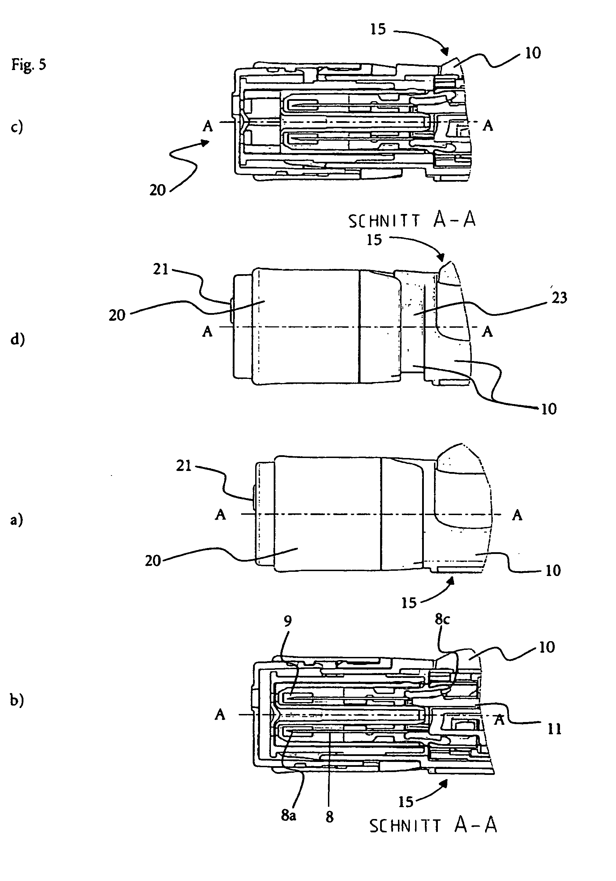

Die Figuren 5a) bis 5d) zeigen das in eine Stechhilfe (15) eingebaute Lanzettenmagazin (7) mit aufgesetzter Fingerkappe (20) einmal in unverlängertem Zustand des Lanzettenmagazins (7) in Fig. 5a) und 5b) sowie in verlängertem Zustand des Lanzettenmagazins (7) in den Fig. 5c) und 5d). In Fig. 5a) ist die Stechhilfe (15) mit regelgerecht aufgebrachter Fingerkappe (20) zu sehen, wobei die Fingerkappe (20) mit dem Gehäuse (10) der Stechhilfe (15) so verrastet ist, dass sie optisch eine Einheit bilden. Ein Schnitt entlang der Schnittlinie A-A dieser zusammengebauten Stechhilfe (15) ist in Fig. 5b) gezeigt. Hier ist wiederum zu sehen, dass das Lanzettenmagazin (7) so mit der Stechhilfe (15) wechselwirkt, dass der Stößel (11) der Stechhilfe (15) mit dem Lanzettenkörperarm (8c) in Kontakt gebracht werden kann, sodass die Lanzette (8) geführt mit ihrer Spitze (8a) aus der Öffnung (21) der Stechhilfe (15) bzw. der Fingerkappe (20) austreten kann.

In Fig. 5b) und 5c) ist der Zustand gezeigt, in dem ein verlängertes Lanzettenmagazin (7) in eine Stechhilfe (15) eingebracht ist, wobei das Magazin (7) zwar mit der Stechhilfe (15) zusammenwirken kann, jedoch die Fingerkappe (20) nicht mehr regelgerecht auf die Stechhilfe aufgesetzt werden kann. Dies ist besonders gut in Fig. 5d) zu erkennen, da die Vertiefung in dem Gehäuse (10) der Stechhilfe (15) offen liegt und nicht von der Fingerkappe (20) erreicht wird, sodass nur eine lose Verbindung zwischen Fingerkappe (20) und der Stechhilfe (15) erreicht wird, die nicht hält, wenn die Stechhilfe (15) alleine an ihrem Gehäuse (10) gefasst wird und in Richtung des distalen Ende (7a) gehalten wird. In diesem Zustand ist für den Benutzer ersichtlich, dass das Magazin (7) nicht regelgerecht mit der Stechhilfe (15) inklusive Fingerkappe (20) zusammensetzbar ist und folglich benutzt sein muss.

### Bezugszeichenliste

- 1: Gehäuse des Lanzettenmagazins
- 2: Verlängerungseinheit
- 2a: Greifkante
- 2b: Kerbe an Verlängerungseinheit
- 3: Kavität
- 3a: Kavitätsbegrenzung
- 4: Verrastungsarm
- 4a: Verrastungsarm innen
- 4b: Kerbe an Verrastungsarm
- 5: Seitenschlitz
- 5a: distales Ende Seitenschlitz
- 5b: proximales Ende Seitenschlitz
- 6: Führung
- 7: Lanzettenmagazin
- 7a: distales Ende (Lanzettenmagazin)
- 7b: proximales Ende (Lanzettenmagazin)
- 8: Lanzette
- 8a: Lanzettenspitze
- 8b: Lanzettenkörper
- 8c: Lanzettenkörperarm
- 9: Sterilschutz
- 10: Gehäuse der Stechhilfe
- 11: Stößel
- 12: Dorn
- 13: Nut
- 15: Stechhilfe
- 20: Fingerkappe
- 21: Öffnung der Fingerkappe
- 23: Vertiefung
- 30: proximale Öffnung des Lanzettenmagazins
- 40: distale Öffnung des Lanzettenmagazins
- 50: optische Markierung

## Patentansprüche

1. Lanzettenmagazin, zur Verwendung in einer Stechhilfe, beinhaltend:
■ ein Gehäuse, mit einem distalen und einem proximalen Ende, mit:
i. mindestens einer Kavität für die Bevorratung von Lanzetten,
ii. mehreren Lanzetten mit jeweils einem Lanzettenkörper am proximalen Ende der Lanzette und einer Lanzettenspitze am distalen Ende der Lanzette, wobei mindestens eine Lanzette so ausgerichtet ist, dass das proximale Ende der Lanzette in Richtung des proximalen Endes des Gehäuses weist und das distale Ende der Lanzette in Richtung des distalen Endes des Gehäuse ausgerichtet ist, und die Lanzetten in unbenutztem Zustand vollständig von dem Gehäuse umgeben sind,
■ eine Verlängerungseinheit, die beweglich mit dem Gehäuse der Lanzettenmagazins verbunden ist,
**dadurch gekennzeichnet, dass** das Gehäuse durch die Verlängerungseinheit in Richtung des distalen Endes des Gehäuses verlängerbar ist, sodass ein Austreten der Lanzette aus dem Lanzettenmagazin verhindert wird, wobei die Verlängerungseinheit nach Bewegen in distaler Richtung relativ zum Lanzettenmagazingehäuse mit dem Gehäuse wechselwirkt, sodass sie manuell nicht wieder in proximale Richtung des Gehäuses bewegbar ist.

2. Lanzettenmagazin nach Anspruch 1, **dadurch gekennzeichnet, dass** die Lanzetten in dem Lanzettenmagazin durch Sicherungsmittel gegen eine Bewegung relativ zum Lanzettenmagazingehäuse durch unbeabsichtigte Krafteinwirkung gesichert sind.

3. Lanzettenmagazin nach Anspruch 2, **dadurch gekennzeichnet, dass** das Sicherungsmittel eine Kunststoffummantelung um die Lanzettenspitze ist, die bei ausreichendem Krafteintrag durch eine Stechhilfe durchstechbar ist.

4. Lanzettenmagazin nach einem der vorherigen Ansprüche, **dadurch gekennzeichnet, dass** das Bewegen der Verlängerungseinheit automatisch bei Entfernen des Magazins aus der Stechhilfe eintritt.

5. Lanzettenmagazin nach einem der vorherigen Ansprüche, **dadurch gekennzeichnet, dass** das Magazin auch in verlängertem Zustand in die Stechhilfe einlegbar ist.

6. Lanzettenmagazin nach einem der vorherigen Ansprüche, **dadurch gekennzeichnet, dass** die Verlängerungseinheit per Hand durch den Benutzer an dem Lanzettenmagazin entlang bewegt wird, wenn er händisch das Magazin aus der Stechhilfe herausnimmt.

7. Lanzettenmagazin nach einem der vorherigen Ansprüche, **dadurch gekennzeichnet, dass** die Verlängerungseinheit eine optische Markierung besitzt, sodass die Markierung sichtbar wird, wenn die Verlängerungseinheit in Richtung distalem Ende des Gehäuses verschoben ist.

8. Lanzettensystem zur Ausführung eines Stechvorgangs in einen Körper, beinhaltend:
■ Eine Stechhilfe mit einem Gehäuse und einem Antriebsmechanismus für eine Lanzette sowie einer Aufnahmeöffnung für ein Lanzettenmagazin
■ Ein Lanzettenmagazin nach einem der Ansprüche 1 bis 7 mit einem Gehäuse, das ein distales und ein proximales Ende aufweist, beinhaltend mindestens eine Lanzette, wobei die Lanzette an ihrem distalen Ende eine Spitze aufweist und mit dem distalen Ende in Richtung distalem Ende des Lanzettenmagazins weist,
■ eine Verlängerungseinheit, die verschiebbar mit dem Gehäuse des Lanzettenmagazins verbunden ist,
**dadurch gekennzeichnet, dass** das Lanzettenmagazin durch die Verlängerungseinheit in Richtung des distalen Endes des Gehäuses verlängerbar ist, sodass ein Austreten der Lanzetten aus dem Lanzettenmagazin bei Wechselwirkung mit der Stechhilfe verhindert wird.

9. Lanzettensystem nach Anspruch 8, **dadurch gekennzeichnet, dass** die Stechhilfe eine Kappe beinhaltet, die abnehmbar mit dem Gehäuse der Stechhilfe verbindbar ist, wobei die Kappe nicht regelgerecht mit dem Gehäuse der Stechhilfe verbindbar ist, wenn die Verlängerungseinheit in Richtung des distalen Endes des Gehäuses des Lanzettenmagazins bewegt ist.

10. Verfahren zur Vermeidung einer Wiederverwendung eines Lanzettenmagazins mit den Schritten:
■ Einlegen eines unbenutzten Lanzettenmagazins mit mindestens einer Lanzette nach Anspruch 1 in eine Stechhilfe,
■ Entfernen des Lanzettenmagazins aus der Stechhilfe, wobei eine bewegbare Verlängerungseinheit am Lanzettenmagazin in Richtung distalem Ende des Lanzettenmagazins bewegt wird.

11. Lanzettenhalterung zur Fixierung von Lanzetten in einem Lanzettenmagazin, beinhaltend:
■ eine Stechhilfe mit einer Aussparung zur Aufnahme eines Lanzettenmagazins, wobei die Stechhilfe eine Entnahmeposition aufweist, in der ein Antriebselement mit einer der Lanzetten in dem Lanzettenmagazin so wechselwirken kann, dass diese Lanzette für einen Stechvorgang in dem Lanzettenmagazin beweglich gelagert ist,
■ ein Lanzettenmagazin nach einem der Ansprüche 1 bis 7 mit mindestens einer Lanzette,
**dadurch gekennzeichnet, dass** die Stechhilfe weiterhin ein Sicherungselement aufweist, die eine Bewegung der Lanzetten, die nicht in der Entnahmeposition positioniert sind, verhindert.

12. Lanzettenhalterung nach Anspruch 11, **dadurch gekennzeichnet, dass** das Sicherungselement in Form eines Dorns ausgebildet ist, der bei Einlegen des Lanzettenmagazins in die Stechhilfe in das Lanzettenmagazin eindringt.

13. Lanzettenhalterung nach Anspruch 12, **dadurch gekennzeichnet, dass** der Dorn eine Aussparung für ein Antriebselement der Stechhilfe aufweist.

14. Lanzettenhalterung nach einem der Ansprüche 11 bis 13, **dadurch gekennzeichnet, dass** das Sicherungselement eine Kunststoffummantelung um die Lanzettenspitze ist, die bei ausreichendem Krafteintrag durch eine Stechhilfe durchstechbar ist.

## Claims

1. Lancet magazine for use in a lancing aid comprising:
■ a housing with a distal and a proximal end having:
i. at least one cavity for the storage of lancets,
ii. a plurality of lancets each having a lancet body at the proximal end of the lancet and a lancet tip at the distal end of the lancet, wherein at least one lancet is aligned such that the proximal end of the lancet points towards the proximal end of the housing and the distal end of the lancet is oriented towards the distal end of the housing, and the lancets in an unused state are completely surrounded by the housing,
■ an extension unit which is movably connected to the housing of the lancet magazine,
**characterized in that** the housing can be extended by the extension unit in the direction of the distal end of the housing so that the lancet is prevented from emerging from the lancet magazine, wherein the extension unit interacts with the housing after movement in the distal direction relative to the lancet magazine housing in such a manner that it cannot again be moved manually in the proximal direction of the housing.

2. Lancet magazine according to claim 1, **characterized in that** the lancets in the lancet magazine are secured by securing means against movement relative to the lancet magazine housing due to unintentional application of force.

3. Lancet magazine according to claim 2, **characterized in that** the securing means is a plastic sheath around the lancet tip which can be pierced by a lancing aid when sufficient force is exerted.

4. Lancet magazine according to one of the previous claims, **characterized in that** the movement of the extension unit occurs automatically when the magazine is removed from the lancing aid.

5. Lancet magazine according to one of the previous claims, **characterized in that** the magazine can also be inserted into the lancing aid in the extended state.

6. Lancet magazine according to one of the previous claims, **characterized in that** the extension unit is moved by hand by the user along the lancet magazine when he manually removes the magazine from the lancing aid.

7. Lancet magazine according to one of the previous claims, **characterized in that** the extension unit has an optimal mark such that the mark is visible when the extension unit has been moved towards the distal end of the housing.

8. Lancet system for carrying out a lancing operation in a body, comprising:
■ a lancing aid with a housing and a drive mechanism for a lancet as well as a receiving opening for a lancet magazine,
■ a lancet magazine according to one of the claims 1 to 7 with a housing which has a distal and a proximal end comprising at least one lancet, where the lancet has a tip at its distal end and the distal end points towards the distal end of the lancet magazine,
■ an extension unit which is movably connected to the housing of the lancet magazine,
**characterized in that** the lancet magazine can be extended by the extension unit in the direction of the distal end of the housing thus preventing the lancets from emerging from the lancet magazine upon interaction with the lancing aid.

9. Lancet system according to claim 8, **characterized in that** the lancing aid has a cap which can be removably connected to the housing of the lancing aid, wherein the cap cannot be properly connected to the housing of the lancing aid when the extension unit is moved towards the distal end of the housing of the lancet magazine.

10. Method for avoiding a reuse of a lancet magazine comprising the steps:
■ inserting an unused lancet magazine containing at least one lancet according to claim 1 into a lancing aid,
■ removing the lancet magazine from the lancing aid, whereupon a movable extension unit on the lancet magazine is moved towards the distal end of the lancet magazine.

11. Lancet holder for securing lancets in a lancet magazine comprising:
■ a lancing aid with an opening for receiving a lancet magazine, where the lancing aid has a removal position in which a drive element can interact with one of the lancets in the lancet magazine in such a manner that this lancet is movably supported in the lancet magazine for a lancing operation,
■ a lancet magazine according to one of the claims 1 to 7 containing at least one lancet,
**characterized in that** the lancing aid additionally has a securing element which prevents movement of the lancets which are not positioned in the collecting position.

12. Lancet holder according to claim 11, **characterized in that** the securing element is configured in the form of a pin which penetrates into the lancet magazine when the lancet magazine is inserted into the lancing aid.

13. Lancet holder according to claim 12, **characterized in that** the pin has a recess for a drive element of the lancing aid.

14. Lancet holder according to one of the claims 11 to 13, **characterized in that** the securing element is a plastic sheath around the lancet tip which can be pierced by a lancing aid when sufficient force is exerted.

## Revendications

1. Magasin de lancettes, destiné à être utilisé dans un auxiliaire de percement, comportant :
- un boîtier, avec une extrémité distale et une extrémité proximale, avec :
i) au moins une cavité pour le stockage de lancettes,
ii) plusieurs lancettes avec respectivement un corps de lancette à l'extrémité proximale de la lancette et une pointe de lancette à l'extrémité distale de la lancette, au moins une lancette étant orientée de telle sorte que l'extrémité proximale de la lancette s'oriente en direction de l'extrémité proximale du boîtier et l'extrémité distale de la lancette étant orientée en direction de l'extrémité distale du boîtier, et les lancettes non utilisées étant complètement entourées par le boîtier,
- une unité à rallonge, qui est assemblée mobile avec le boîtier du magasin de lancettes,
**caractérisé en ce que** le boîtier peut être rallongé par l'unité à rallonge en direction de l'extrémité distale du boîtier, ce qui empêche que la lancette s'échappe du magasin de lancettes, l'unité à rallonge interagissant avec le boîtier après le déplacement en direction distale par rapport au boîtier de magasin de lancettes, de telle sorte qu'elle ne peut plus être déplacée manuellement en direction proximale du boîtier.

2. Magasin de lancettes selon la revendication 1, **caractérisé en ce que** les lancettes sont sécurisées dans le magasin de lancettes par un moyen de sécurité contre un déplacement par rapport au boîtier de magasin de lancettes sous l'effet d'une force intempestive.

3. Magasin de lancettes selon la revendication 2, **caractérisé en ce que** le moyen de sécurité est une enveloppe en matière plastique autour de la pointe de lancette, qui peut être transpercée par un auxiliaire de percement en cas d'apport de force suffisant.

4. Magasin de lancettes selon l'une des revendications précédentes, **caractérisé en ce que** le déplacement de l'unité à rallonge intervient automatiquement en retirant le magasin hors de l'auxiliaire de percement.

5. Magasin de lancettes selon l'une des revendications précédentes, **caractérisé en ce que** le magasin peut être inséré dans l'auxiliaire de percement également à l'état rallongé.

6. Magasin de lancettes selon l'une des revendications précédentes, **caractérisé en ce que** l'unité à rallonge est déplacée manuellement par l'utilisateur le long du magasin de lancettes quand il retire le magasin hors de l'auxiliaire de percement à la main.

7. Magasin de lancettes selon l'une des revendications précédentes, **caractérisé en ce que** l'unité à rallonge dispose d'un repère visuel, de telle sorte que le repère est visible quand l'unité à rallonge est déplacée en direction de l'extrémité distale du boîtier.

8. Système de lancettes pour réaliser un processus de percement dans un corps, comportant :
- un auxiliaire de percement avec un boîtier et un mécanisme d'entraînement pour une lancette ainsi qu'une ouverture de réception pour un magasin de lancettes,
- un magasin de lancettes selon l'une des revendications 1 à 7 avec un boîtier, qui présente une extrémité distale et une extrémité proximale, comportant au moins une lancette, la lancette présentant une pointe à son extrémité distale et étant orientée avec l'extrémité distale en direction de l'extrémité distale du magasin de lancettes,
- une unité à rallonge, qui est assemblée coulissante avec le boîtier du magasin de lancettes,
**caractérisé en ce que** le magasin de lancettes peut être rallongé par l'unité à rallonge en direction de l'extrémité distale du boîtier, ce qui empêche que les lancettes s'échappent du magasin de lancettes lors de l'interaction avec l'auxiliaire de percement.

9. Système de lancettes selon la revendication 8, **caractérisé en ce que** l'auxiliaire de percement comporte un capuchon, qui peut être assemblé amovible avec le boîtier de l'auxiliaire de percement, le capuchon ne pouvant pas être assemblé correctement avec le boîtier de l'auxiliaire de percement quand l'unité à rallonge est déplacée en direction de l'extrémité distale du boîtier du magasin de lancettes.

10. Procédé pour empêcher la réutilisation d'un magasin de lancettes présentant les étapes suivantes :
- insertion d'un magasin de lancettes non utilisé avec au moins une lancette selon la revendication 1 dans un auxiliaire de percement,
- retrait du magasin de lancettes hors de l'auxiliaire de percement, une unité à rallonge mobile sur le magasin de lancettes étant déplacée en direction de l'extrémité distale du magasin de lancettes.

11. Support de lancettes pour la fixation de lancettes dans un magasin de lancettes, comportant :
- un auxiliaire de percement avec un évidement pour recevoir un magasin de lancettes, l'auxiliaire de percement présentant une position de prélèvement dans laquelle un élément d'entraînement peut interagir avec une des lancettes dans le magasin de lancettes de telle sorte que cette lancette est montée mobile pour un processus de percement dans le magasin de lancettes,
- un magasin de lancettes selon l'une des revendications 1 à 7 avec au moins une lancette,
**caractérisé en ce que** l'auxiliaire de percement présente en outre un élément de sécurité, qui empêche le déplacement des lancettes qui ne sont pas positionnées en position de prélèvement.

12. Support de lancettes selon la revendication 11, **caractérisé en ce que** l'élément de sécurité présente la forme d'un mandrin qui pénètre dans le magasin de lancettes en insérant le magasin de lancettes dans l'auxiliaire de percement.

13. Support de lancettes selon la revendication 12, **caractérisé en ce que** le mandrin présente un évidement pour un élément d'entraînement de l'auxiliaire de percement.

14. Support de lancettes selon l'une des revendications 11 à 13, **caractérisé en ce que** l'élément de sécurité est une enveloppe en matière plastique autour de la pointe de lancette, qui peut être transpercée par un auxiliaire de percement en cas d'apport de force suffisant.
